# FASCICULE DE BREVET EUROPEEN

(11) **EP 4 041 329 B1**
(45) Date de publication et mention de la délivrance du brevet: **19.07.2023**
(21) Numéro de dépôt: 20797818.0
(22) Date de dépôt: 09.10.2020
(51) Int. Cl.: A61L 31/04, A61L 31/08

(54) **PROCEDE DE MINERALISATION D'UNE MEMBRANE DE BIOPOLYMERE ET MEMBRANES AINSI OBTENUES**
VERFAHREN ZUR MINERALISIERUNG EINER BIOPOLYMERMEMBRAN UND RESULTIERENDE MEMBRANEN
METHOD FOR MINERALIZING A BIOPOLYMER MEMBRANE, AND RESULTING MEMBRANES

(30) Priorité: 10.10.2019 FR 1911259
(43) Date de publication de la demande: 17.08.2022
(73) Titulaire: Regeska, 69001 Lyon (FR)
(72) Inventeur: LITVINOV, Sergei, SAMARA, 443079 (RU)
(74) Mandataire: Regimbeau
(86) Numéro de dépôt international: PCT/FR2020/051783
(87) Numéro de publication internationale: WO 2021/069846

(56) Documents cités:
- WO-A2-2013/190534
- RU-C1- 2 410 040
- US-A- 5 532 217
- US-B1- 6 395 036

## Description

### DOMAINE DE L'INVENTION

La présente invention concerne le domaine du génie tissulaire, c'est-à-dire la fabrication de tissus organiques synthétiques biocompatibles, pouvant être implantés dans des organismes vivants.

Plus particulièrement, la présente invention concerne un procédé de préparation d'un biomatériau pour un usage en chirurgie et médecine humaine. Ce biomatériau consiste en une membrane de biopolymère recouverte de nanocristaux de composés minéraux, présentant une structure particulière. Cette membrane de biopolymère peut notamment être recouverte de nanocristaux de composés phosphocalciques, notamment choisis parmi de l'hydroxyapatite ou de la brushite.

### ETAT DE LA TECHNIQUE

En médecine, l'implantation de structures de biopolymère dans des organismes vivants est une technique classique, utilisée pour favoriser la reconstruction de tissus biologiques, notamment la reconstruction des tissus osseux.

Ces structures de biopolymère, utilisées comme substituts osseux, sont composées de fibres naturelles (sous la forme de films, fibres, structures compactes et/ou poreuses) souvent associées avec des composés actifs pour améliorer leur biocompatibilité : des cellules, des hormones et/ou facteurs de croissance, ou encore des minéraux.

Ces substituts osseux doivent permettre l'ostéoconduction, qui désigne la capacité du biomatériau à servir de support passif à la repousse osseuse. Le biomatériau doit en outre être poreux et résorbable. La porosité du biomatériau permet à l'implant de se vasculariser et se résorber progressivement. La vitesse de résorption du biomatériau doit correspondre aux besoins de la néoformation osseuse : pas trop vite, pour qu'il puisse servir de support à la formation osseuse ; pas trop lentement, pour limiter les risques d'infection et pour ne pas gêner la croissance de l'os par encombrement mécanique.

Plusieurs types de biomatériaux sont utilisés comme support passif de colonisation cellulaire et tissulaire : il peut s'agir de céramiques naturelles ou synthétiques, ou encore de différents matériaux d'origine naturelle, dont la composition chimique se rapproche de celle de la phase minérale de l'os.

Les biomatériaux synthétiques les plus souvent utilisés pour le comblement des défauts osseux sont l'hydroxyapatite et les phosphates tricalciques, deux espèces minérales de la famille des phosphates, purs ou en mélange.

La combinaison de biopolymère et d'hydroxyapatite nanocristalline, telle que collagène et hydroxyapatite, est connue et utilisée comme substitut d'os depuis les années 1990 (voir par exemple US 5,231,169, US 6,201,039, EP0747067).

De nombreuses applications thérapeutiques de ce biomatériau ont été proposées, par exemple :
- La demande de brevet EP 3181158 est relative à un biomatériau nanoporeux, comprenant de l'hydroxyapatite déposée ou précipitée sur un gel de polymère biodégradable, choisi notamment parmi le collagène et l'alginate. Ce biomatériau peut être utilisé en orthopédie, pour la chirurgie dentaire et la chirurgie reconstructive.
- La demande internationale WO 2008/096334 décrit un implant destiné à réparer une lésion du cartilage, comprenant un squelette composé de collagène et d'hydroxyapatite.
- La demande de brevet RU2410040 décrit une prothèse composée d'une membrane de collagène ou d'alginate, recouverte de cristaux d'hydroxyapatite, destinée au traitement chirurgical de l'incontinence urinaire chez la femme.
- La demande internationale WO 2013/111077 décrit une composition comprenant, entre autres, de l'alginate de sodium et de l'hydroxyapatite, pour le traitement des troubles gastro-intestinaux.
- La demande internationale WO 2013/190534 décrit une poudre composite compactable comprenant une composition de silicate, collagène et phosphate de calcium. Cette poudre peut être obtenue par broyage d'un matériau composé de collagène minéralisé, et peut être utilisée comme implant. Plusieurs procédés de minéralisation de membranes de biopolymère ont été décrits. Des techniques classiques consistent à cuire les minéraux à des températures très élevées, comme de la céramique, ce qui engendre des cristaux extrêmement durs.

Des techniques plus « douces » ont également été proposées :
- Le brevet US 6,395,036 décrit un procédé de préparation d'un matériel composite, où des ions calcium et des ions phosphate circulent autour d'une membrane de collagène et précipitent, lorsqu'ils se rencontrent, en cristaux d'hydroxyapatite.
- La demande internationale WO 2008/096334 décrit un procédé de préparation d'un échafaudage en collagène, recouvert de cristaux d'hydroxyapatite, par lyophilisation et gel.

- Le brevet EP 3021883 décrit un matériau composite comprenant un squelette de fibres de collagène, au moins partiellement revêtues de nanocristaux d'hydroxyapatite à croissance épitactique. Le procédé d'obtention comprend l'immersion de collagène fibreux dans une solution aqueuse saturée d'ions calcium et phosphate (Ca2+/HxPO4(3-x)), des nanocristaux d'hydroxyapatite se formant sur les fibres de collagène, le procédé étant arrêté par retrait du collagène de la solution, et rinçage.
- Le brevet RU2174848 décrit une méthode de préparation d'une membrane de collagène minéralisée, comprenant l'utilisation d'électrodes pour faire circuler les ions par électrophorèse. Sous l'action de ce courant électrique, la solution contenant des cations calcium s'acidifie, en créant ainsi des ions hydrogène susceptibles de dissoudre les cristaux d'hydroxyapatite formés. La solution proposée est de stabiliser le pH des solutions à 11 pour la solution d'ions calcium, et à une valeur de 10.5 à 11 pour celle contenant les ions phosphate, en ajoutant continuellement de l'hydroxyde de calcium à la solution contenant les ions calcium.
- Les brevets US 5,532,217 et US 5,739,286 sont relatifs à un procédé de minéralisation de fibres de collagène, dans lequel les fibres de collagène sont insérées dans un récipient comprenant deux réservoirs, l'un contenant du chlorure de calcium, et l'autre du phosphate de potassium. Les deux récipients sont séparés par un tube constitué de membranes de dialyse en cellulose, dans lequel les fibres de collagène sont insérées. Les pH des solutions sont ajustés de manière à ce que les ions précipitent, en formant des nanocristaux sur le collagène. La procédure, relativement lente, dure 7 jours.
- Le brevet US 6,589,590 décrit un procédé de minéralisation d'un biopolymère, comprenant la mise en contact d'une membrane de biopolymère avec, d'un côté une solution contenant des ions calcium, et de l'autre côté une solution contenant des ions phosphate ; les ions diffusant à travers la membrane de biopolymère précipitent lors de leur rencontre en cristaux d'hydroxyapatite.

La présente invention est relative à un nouveau procédé de minéralisation « douce » d'une membrane de biopolymère, mettant en oeuvre une combinaison originale de différents procédés afin d'obtenir des nanocristaux de minéraux sur ladite membrane. Cette membrane minéralisée présente des caractéristiques nouvelles intéressantes. En effet, les cristaux formés sont dans un état de faible cristallinité et avec des qualités structurelles particulières. La membrane minéralisée présente une résorption rapide (entre 5 et 60 jours, et en particulier en moins de 30 jours, en moins de 25 jours, voire moins de 21 jours) après implantation dans un organisme vivant, et a des effets biologiques positifs (anti-oxydants, anti-inflammatoires) dès l'implantation dans l'organisme, ou après administration d'une poudre préparée à partir de cette membrane minéralisée.

Les propriétés nouvelles de ces membranes de biopolymère minéralisées sont telles que de nouvelles applications thérapeutiques peuvent être envisagées, en sus du traitement des désordres osseux et cartilagineux.

### EXPOSE DE L'INVENTION

L'étendue de la protection est définie par les revendications. Les modes de réalisation se rapportant à une méthode thérapeutique ne sont pas revendiqués.

La présente invention concerne un procédé de minéralisation d'une membrane de biopolymère, comprenant les étapes suivantes :
a) Introduction d'un assemblage (3) constitué d'une membrane de biopolymère (4) comprise entre deux feuilles de cellulose (A) et (B), dans un récipient comprenant :
   - un premier compartiment (1) et un second compartiment (2), comprenant chacun une électrode, une première électrode étant une anode placée dans le premier compartiment (1) et une seconde électrode étant une cathode placée dans le second compartiment (2),
   - les parois du premier compartiment (1) et du second compartiment (2) mises en contact l'une avec l'autre présentant chacune une ouverture mettant en communication le premier et le second compartiment,
   l'assemblage (3) étant disposé dans ladite ouverture entre le premier et le second compartiment de manière à l'obturer, la feuille (A) de cellulose étant du côté du premier compartiment (1) et la feuille (B) de cellulose du côté du deuxième compartiment (2),
b) remplissage du premier compartiment (1) avec une solution aqueuse contenant au moins un cation choisi parmi : des ions calcium, des ions argent, des ions zinc, des ions cuivres, des ions sodium, des ions magnésium et des ions aluminium, et du second compartiment (2) avec une solution aqueuse contenant au moins un anion choisi parmi des ions fluorure, des ions sulfates, des ions carbonates, des ions silicates et des ions phosphate ;
c) Application d'une tension électrique entre les électrodes ;
d) retournement de l'assemblage (3) de manière à ce que la feuille (A) de cellulose soit du côté du second compartiment et la feuille (B) de cellulose du côté du premier compartiment, ou échange des solutions et électrodes du premier et du second compartiment;
   c') application d'une tension électrique entre les électrodes, ladite tension étant égale à celle appliquée à l'étape (c) et étant appliquée pendant une durée identique à celle de l'étape (c) ;
e) retrait et rinçage de l'assemblage (3) ;
f) récupération et séchage de la membrane de biopolymère minéralisée.

La présente invention concerne également une membrane de biopolymère minéralisée sur ses deux faces, susceptible d'être obtenue par le procédé décrit ladite membrane de biopolymère étant minéralisée sur ses deux faces avec des nanocristaux de phosphate de calcium organisés sous forme de plaquettes et de cristaux nanoporeux sphériques présentant une structure alvéolaire.

Un autre objet de l'invention est une poudre constituée d'une membrane de biopolymère minéralisée telle que décrite ci-dessus, réduite en poudre.

Un autre objet de l'invention est une solution comprenant ladite poudre en suspension dans un véhicule pharmaceutiquement acceptable.

La présente invention concerne également une membrane de biopolymère minéralisée telle que décrite, ou une poudre telle que décrite, ou une solution telle que décrite, pour son utilisation thérapeutique dans le traitement des désordres osseux, cartilagineux, pancréatiques, rénaux, urétraux, urétéraux et vésicaux, testiculaires, ovariens, intestinaux, hépatiques, neurologiques, cardiaques, tympaniques, oculaires, urinaires, gynécologiques, pulmonaires, bronchiques, trachéaux, vasculaires, conjonctifs, cutanés, muqueux, dentaires, gingivaux et/ou du tissu hématopoïétique et immunitaire.

### DESCRIPTION DES FIGURES

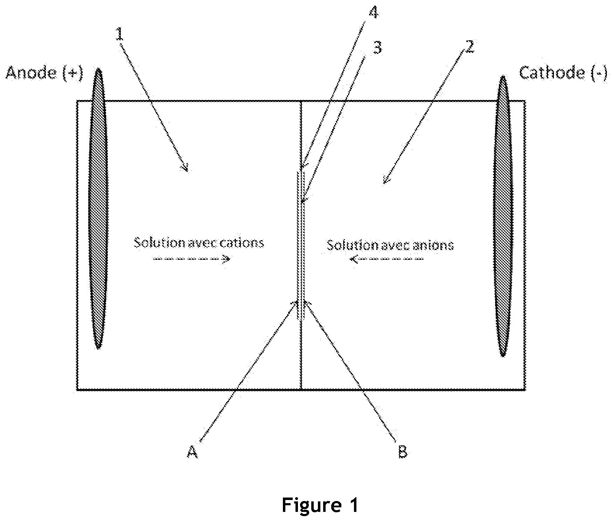

Figure 1. La figure 1 représente une coupe transversale d'un récipient comprenant un premier compartiment (1) et un second compartiment (2), comprenant chacun une électrode, l'anode étant placée dans le premier compartiment (1) et la cathode dans le second compartiment (2), et un assemblage (3) disposé dans l'ouverture entre le premier et le second compartiment de manière à l'obturer. Cet assemblage est constitué d'une membrane de biopolymère (4) entourée de deux feuilles de cellulose, la feuille (A) étant du côté du premier compartiment (1) et la feuille (B) de cellulose du côté du deuxième compartiment, lors de l'étape (c).
Figure 2. La figure 2 représente le même dispositif que la figure 1 mais lors de l'étape (c'), l'assemblage (3) ayant été retourné de manière à ce que la feuille (A) de cellulose soit du côté du second compartiment (2) et la feuille (B) de cellulose du côté du premier compartiment (1).
Figure 3. La figure 3 représente la variation d'activité antioxydante totale mesurée dans les urines d'un individu (Homme âgé de 67 ans, en bonne santé) auquel a été administré une poudre selon l'invention. L'activité anti-oxydante totale (TAC) est exprimée en pourcentage par rapport aux valeurs témoins, en fonction du nombre de jours d'administration (en abscisse).
Figure 4. Photos des cristaux présents sur les membranes, par microscopie électronique à balayage (MEB) Zeiss Supra 55VP, sur les deux faces des membranes de collagènes minéralisées. Les échantillons 7, 4, 6 et 5 ont été photographiés avec les grossissements suivants (de gauche à droite) : x250, x750, x2000, x10.000, x20.000 et x30.000. Tout à gauche, une photo sans grossissement montre l'aspect de la membrane minéralisée. L'échantillon 5 correspond à la membrane obtenue par le procédé selon l'invention.
Figure 5. Photos des cristaux présents sur la membrane de l'échantillon 5, par microscopie électronique à balayage (MEB) Zeiss Supra 55VP, aux grossissements suivants : x20.000 et x30.000. La face 1 est en haut, la face 2 en bas.
Figure 6 : Diffractogrammes des matrices de collagène minéralisées. Comparaison des spectres de diffraction obtenus sur les échantillons 7, 4, 6 et 5 broyés manuellement.
Figure 7. *Curve fitting* dans le domaine des HPO₄ sur spectres IR normalisés. Comparaison des bandes relatives aux groupement PO₄ et HPO₄ apatitique et HPO₄ labile sur les échantillons 5 (en haut) et 7 (en bas) broyés manuellement.

### DESCRIPTION DETAILLEE DE L'INVENTION

La présente invention concerne un procédé de minéralisation d'une membrane de biopolymère, permettant d'obtenir des nanocristaux d'un type particulier sur cette membrane. La membrane présente l'avantage d'une résorption rapide après implantation dans un organisme vivant, et a des effets biologiques positifs dès son implantation dans l'organisme, ou suite à administration d'une poudre obtenue à partir de ladite membrane.

### Membrane de biopolymère

Une membrane désigne dans le langage commun une paroi souple. Chaque membrane présente une rigidité et une porosité spécifique.

Un polymère est une macromolécule constituée d'un assemblage de nombreuses unités monomériques. Un biopolymère désigne un polymère naturel, directement produit par des êtres vivants (végétaux, algues, animaux, champignons, etc.) ou issu directement d'un produit naturel. Ils sont composés d'acides aminés, de sucres, de nucléotides ou encore d'autres composés monomériques organiques. On distingue les biopolymères d'origine végétale et ceux d'origine animale.

Parmi les biopolymères d'origine végétale, on citera notamment la cellulose, l'amidon, et l'alginate (produit par des algues).

Parmi les biopolymères d'origine animale, on citera notamment le collagène et le chitosan, issu du traitement de la chitine produite par les crustacés.

Selon une mise en oeuvre préférée de l'invention, la membrane de biopolymère est une membrane de collagène, d'alginate ou de chitosan.

Selon une première mise en oeuvre, la membrane est constituée de collagène.

Le collagène est une macromolécule protéique, constituée de trois chaînes alpha polypeptidiques associées, constituées d'acides aminés. Ces chaînes sont reliées entre elles par des liaisons hydrogène entre l'hydroxylysine et l'hydroxyproline et des liaisons covalentes. La macromolécule est organisée le plus souvent sous forme de fibres. Plusieurs types d'association de chaînes polypeptidiques et de configuration sont possibles ; ainsi, il existe plusieurs types de collagène dénommés type I, type II, type II, etc. Le collagène est présent dans la matrice extracellulaire des cellules animales, et confère aux tissus une résistance mécanique à l'étirement. Le collagène est très peu allergénique, et est utilisé dans de nombreuses applications thérapeutiques.

Selon une deuxième mise en oeuvre, la membrane est constituée d'alginate.

L'alginate est un polysaccharide produit par des algues. Il s'agit d'un polymère formé de deux monomères: le mannuronate ou acide mannuronique, dont certaines unités sont acétylées, et le guluronate ou acide guluronique. La proportion et la distribution de ces deux monomères varient : il existe donc plusieurs types d'alginates, ayant des propriétés chimiques et physiques variables. En médecine, l'alginate est utilisé pour encapsuler des médicaments ou des substances biologiques fragiles. L'alginate peut aussi être utilisé dans la confection de certains pansements. C'est aussi le produit utilisé par les dentistes pour les prises d'empreintes dentaires.

Selon une troisième mise en oeuvre, la membrane est constituée de chitosan.

Le chitosan (aussi dénommé chitosane) est un polyoside composé de la distribution aléatoire de D-glucosamine liée en β-(1-4) (unité désacétylée) et de N-acétyl-D-glucosamine (unité acétylée). Il est produit par désacétylation chimique en milieu alcalin ou enzymatique de la chitine, le composant de l'exosquelette des arthropodes, de l'endosquelette des céphalopodes, ou encore de la paroi des champignons. Le chitosan est largement utilisé dans les produits cosmétiques et diététiques. En médecine, le chitosan peut être utilisé en tant que biomatériau, notamment pour la régénération tissulaire, l'hémostase et l'ostéogénèse.

La forme de la membrane de biopolymère sera par exemple un parallélépipède, tel qu'un carré, un rectangle ou un losange.

La taille de la membrane de biopolymère sera choisie par l'Homme du métier, en fonction des applications envisagées ; elle pourra par exemple être d'une taille d'environ 10 cm², ou 20 cm², ou encore 100 cm² (carré de 10*10 cm). Elle pourra également présenter des mesures bien plus amples, par exemple être d'une taille de 1 m², 10 m² ou même 100 m2.

### Assemblage

Pour le procédé de minéralisation d'une membrane de biopolymère selon l'invention, ladite membrane est disposée au sein d'un assemblage constitué de deux feuilles de cellulose identiques, dénommées (A) et (B) pour distinguer leur emplacement vis-à-vis des deux compartiments contenant des solutions aqueuses distinctes.

Cet assemblage est placé entre les deux compartiments (1) et (2), de manière à les séparer hermétiquement : la seule surface de rencontre des deux solutions placées dans chacun des compartiments (1) et (2) étant cet assemblage.

Les feuilles de cellulose (A) et (B) utilisées sont identiques. Elles sont de taille légèrement supérieure à celle de la membrane de biopolymère, afin d'assurer une étanchéité entre le compartiment (1) et le compartiment (2). Leur épaisseur est de préférence de 0,02 mm à 0,03 mm. Il pourra notamment s'agir de feuilles de cellophane.

### Minéralisation des membranes : solutions ioniques utilisées

Lors de l'étape (b) du procédé selon l'invention, le premier et le second compartiment sont remplis avec les solutions aqueuses suivantes :
- Dans le premier compartiment (1), une solution aqueuse contenant au moins un cation choisi parmi : des ions calcium, des ions argent, des ions zinc, des ions cuivre, des ions sodium, des ions magnésium et des ions aluminium, et
- Dans le second compartiment (2), une solution aqueuse contenant au moins un anion choisi parmi des ions fluorure, des ions sulfates, des ions carbonates, des ions silicates et des ions phosphate.

Par exemple, les solutions suivantes pourront être employées pour la mise en oeuvre de l'invention :

**Tableau 1**

| Solutions et/ou cation(s) présents dans la solution du compartiment (1) | Solutions et/ou anion(s)présents dans la solution du compartiment (2) | Cristaux formés sur la membrane de biopolymère |
|---|---|---|
| Chlorure de calcium | Phosphate d'ammonium | Hydroxyapatite |
| Ca(Cl)₂ | NH₄H₂PO₄ | Ca₅(PO₄)₃(OH) |
| | (NH₄)₂HPO₄ | |
| | (NH₄)₃PO₄ | |
| Chlorure de calcium Ca(Cl)₂, additionné d'ions argent (Arg²⁺), et/ou d'ions zinc (Zn²⁺) et/ou d'ions cuivre (Cu⁺ ou Cu²⁺) | Phosphate d'ammonium | Hydroxyapatite substituée avec ions argent, zinc, cuivre, et/ou silicates |
| | NH₄H₂PO₄ | |
| | (NH₄)₂HPO₄ | |
| | (NH₄)₃PO₄, éventuellement additionné d'ions silicates (SiO₄)⁴ | |
| Ions magnésium Mg²⁺ | Ions sulfate SO₄²⁻ | Sulfate de magnésium MgSO₄ |
| Ions aluminium Al³⁺ | Ions sulfate SO₄² | Sulfate d'aluminium Al₂(SO₄)₃ |

Selon une mise en oeuvre préférée de l'invention, la solution aqueuse mise dans le premier compartiment (1) contient au moins des ions calcium, et la solution aqueuse mise dans le second compartiment (2) contient au moins des ions phosphate.

Selon une autre mise en oeuvre préférée de l'invention, la solution aqueuse mise dans le premier compartiment (1) contient comme cations uniquement des ions calcium, et la solution aqueuse mise dans le second compartiment (2) contient comme anions uniquement des ions phosphate, en sus de la présence des ions issus de la décomposition de l'eau.

Préférentiellement, il s'agira des solutions suivantes :
- chlorure de calcium Ca(Cl)₂ additionné d'hydroxyde de calcium Ca(OH)₂, et de
- phosphate d'ammonium, avec une, deux ou trois solutions de phosphate d'ammonium substituées, additionné d'une solution d'ammoniac (NH3) concentrée à 25% en masse.

Il est entendu que ces solutions pourront comprendre, outre les ions précisés ci-dessus, d'autres ions permettant d'optimiser la réaction chimique de minéralisation de la membrane de biopolymère.

Par exemple, il pourra être nécessaire d'ajuster le pH de chacune des solutions, en ajoutant un acide ou une base.

Selon une mise en oeuvre préférée, le pH de la solution aqueuse contenant au moins un anion est compris entre 7 et 11. Ceci est notamment obtenu par l'addition, dans la solution aqueuse, d'une solution concentrée d'ammoniac (NH₃).

Selon une autre mise en oeuvre préférée, de l'hydroxyde de calcium est ajouté à la solution de chlorure de calcium afin de maintenir un taux d'ions calcium constant, ceci permettant d'éviter une trop forte acidification de la solution de chlorure de calcium.

En fonction des solutions aqueuses utilisées, par exemple pour les solutions contenant des ions zinc ou argent ou magnésium ou aluminium, l'Homme du métier adaptera en fonction des besoins les autres composés à ajouter auxdites solutions pour maintenir un pH spécifique et/ou optimiser la réaction.

Avantageusement, pour que la réaction soit la plus efficace possible, chacune des solutions utilisées sera protégée de toute contamination par l'autre solution aqueuse.

Selon une mise en oeuvre du procédé de l'invention, les solutions sont versées dans chacun des compartiments (1) et (2) après installation de l'assemblage (3), de manière à éviter toute contamination.

### Dispositif : récipient et électrodes

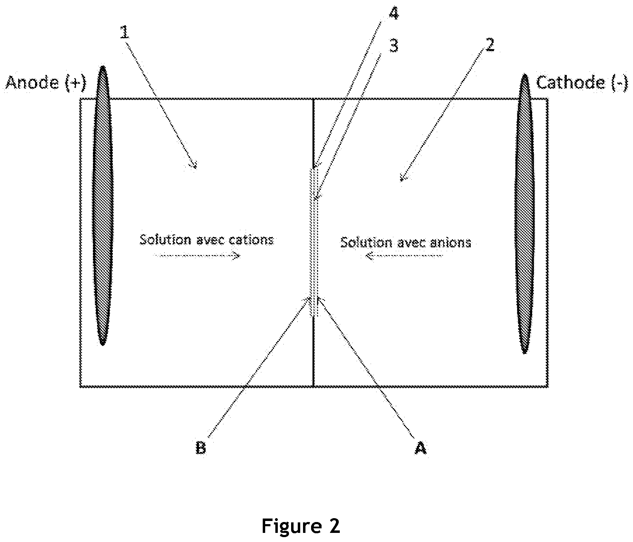

Un dispositif adapté pour la mise en oeuvre du procédé selon l'invention est représenté schématiquement dans les figures 1 et 2. Il est désigné par l'appellation « bac à électrophorèse » et inclut le récipient et les électrodes.

Ce dispositif comprend un premier compartiment (1) et un second compartiment (2), comprenant chacun une électrode, l'anode étant placée dans le premier compartiment (1) et la cathode dans le second compartiment (2) pour la mise en oeuvre de l'étape (c) du procédé.

L'anode est définie comme étant le pôle d'attraction des ions négatifs (anions) soumis à un champ électrique dans un processus d'électrolyse. Elle peut être par exemple constituée de fibres de carbone ou de graphite. Les éléments de connexion peuvent avantageusement être en or ou platine.

La cathode est définie comme étant le pôle d'attraction des ions positifs (cations) soumis à un champ électrique dans un processus d'électrolyse. Elle peut être par exemple en acier.

Le dispositif peut être constitué de tout type de matériau solide inerte, tel que du verre, du plastique ou du plexiglas.

Avantageusement, chaque compartiment possède un système de vidange indépendant, ce qui facilite la réalisation de l'étape (d) d'échange des solutions et électrodes entre les deux compartiments.

Le dispositif comprend également une ouverture de chacune des parois du premier compartiment (1) et du second compartiment (2) qui sont mises en contact l'une avec l'autre, ce qui permet la mise en communication du premier et du second compartiment.

Ces ouvertures, également désignées « fenêtres », auront une taille adapte à celle de la membrane de biopolymère (4). Elles pourront par exemple présenter une surface de 10 cm x 10 cm, ou de 15 cm x 15 cm.

Selon une mise en oeuvre préférée de l'invention, le dispositif est équipé d'un agitateur, notamment plongé dans la solution du compartiment (1) contenant au moins un cation, afin d'homogénéiser en permanence ladite solution.

### Etapes (c) et (c') : application d'une tension électrique entre les électrodes

Lors de ces deux étapes, une tension électrique (courant continu) est appliquée entre les deux électrodes par le biais d'un générateur électrique.

De manière préférée, l'intensité est réglée de manière à obtenir une tension réelle de 2 à 13 Volts, ou de 3 à 13 Volts, ou mieux de 5 à 12 Volts, ou encore de 7 à 10 Volts.

La tension réelle est identique lors des deux étapes (c) et (c'). Elle est également appliquée pendant une durée égale lors des deux étapes, ceci permettant d'obtenir le même niveau de minéralisation sur chaque face de la membrane de biopolymère.

Il est à noter que la tension varie au cours de l'étape : pour une intensité de courant programmée, la tension va baisser spontanément lorsque la conductivité est bonne. De plus, l'intensité chute en fin de processus car les nanocristaux formés freinent le passage du courant électrique.

Cette tension électrique est appliquée, lors des étapes (c) et (c'), pendant une durée de 8 à 16 heures, préférentiellement pendant environ 12 heures.

Sous l'effet de cette tension électrique, les ions vont se déplacer dans les solutions entourant l'assemblage, et après avoir traversé les feuilles de cellulose (A) et (B), se déposer sur la membrane de biopolymère en formant des nanocristaux, plutôt du côté de la solution contenant les cations.

De manière préférée, lors des étapes (c) et (c'), la solution aqueuse contenant au moins un cation est agitée en continu, pour maintenir un pH basique et éviter la dissolution des cristaux formés par la genèse d'ions H⁺.

De manière préférée, lors des étapes (c) et (c'), la solution aqueuse contenant au moins un cation est saturée en cations par un ajout continu d'hydroxyde dudit cation, ceci permettant de maintenir le pH de la solution contenant des ions calcium à une valeur proche de 11 ; de plus cela permet de reconstituer de manière constante des cations. Les avantages de cette mise en oeuvre sont présentés dans le brevet RU2174848.

### Etape (d) : retournement de la membrane de biopolymère

L'étape (d) permettant de minéraliser l'autre face de la membrane peut être réalisée selon deux modalités différentes :
1) soit l'assemblage (3) constitué de la membrane de biopolymère et des feuilles de cellulose est retiré de l'ouverture entre les deux compartiments, et est retourné de manière à ce que la feuille (A) soit désormais en contact avec la solution aqueuse du second compartiment, et la feuille (B) en contact avec la solution aqueuse du premier compartiment ; naturellement, toutes les précautions sont prises pour éviter la contamination de chaque solution avec des ions issus de l'autre solution.
2) soit un échange des solutions et des électrodes du premier et du second compartiment est réalisé. Ainsi la cathode est placée dans le premier compartiment (1), dans une solution contenant au moins un anion, et l'anode dans le second compartiment (2), dans une solution contenant au moins un cation, pour la réalisation de l'étape (c') telle que décrite ci-dessus.

Selon une première mise en oeuvre du procédé selon l'invention, l'étape (d) est la suivante : retournement de l'assemblage (3) de manière à ce que la feuille (A) de cellulose soit du côté du second compartiment et la feuille (B) de cellulose du côté du premier compartiment.

Selon une seconde mise en oeuvre du procédé selon l'invention, l'étape (d) est la suivante : échange des solutions et électrodes du premier et du second compartiment.

### Etapes finales (e) et (f) du procédé

A l'issue de l'étape (c'), la tension électrique est arrêtée ; et l'assemblage (3) est retiré puis rincé à l'eau. Les feuilles de cellulose (A) et (B) sont retirées et jetées.

La membrane de biopolymère minéralisée sur ses deux faces est séchée, par toute technique connue de l'Homme du métier.

Avantageusement, l'étape (f) de séchage de la membrane de biopolymère minéralisée est effectuée à une température inférieure à 35°C.

De préférence, l'étape de séchage dure de 12 à 24 heures.

### Membrane de biopolymère minéralisée

Un des objets de la présente invention est une membrane de biopolymère minéralisée sur ses deux faces, susceptible d'être obtenue par le procédé tel que décrit dans la présente demande.

Un autre objet de l'invention est une membrane de biopolymère, minéralisée sur ses deux faces, obtenue par le procédé décrit dans la présente demande.

Cette membrane de biopolymère est caractérisée en ce qu'elle comporte, sur ses deux faces, des nanocristaux organisés sous forme de plaquettes et de cristaux nanoporeux sphériques présentant une structure alvéolaire.

Ces nanocristaux pourront notamment être constitués des composés minéraux suivants :
- Des composés phosphocalciques, notamment choisis parmi les suivants :
   ∘ De l'hydroxyapatite ; et/ou
   ∘ De l'hydroxyapatite déficiente dopée avec des ions fluorures, argent, ou zinc ; et/ou
   ∘ Une apatite poly-substituée ou une hydroxy, fluoro ou carboxy-apatite déficiente ; et/ou
   ∘ De la brushite (phosphate dicalcique hydraté : CaHPO₄.2H₂O) ; et/ou
   ∘ Des composés phosphocalciques avec des groupements hydrogéno-phosphate labiles,
- du sulfate de magnésium, ou encore
- du sulfate d'aluminium, ou
- toute combinaison de ces composés minéraux

La précipitation des ions sur la membrane de biopolymère génère la formation de nanocristaux, définis comme étant des cristaux d'une taille de l'ordre du nanomètre (de l'ordre de 43 à 45 nm). Ces nanocristaux forment ensuite des agglomérats, d'une taille micrométrique.

Selon une mise en oeuvre particulière, cette membrane est recouverte sur ses deux faces de nanocristaux de phosphate de calcium, également désignés nanocristaux de composés phosphocalciques.

Selon un aspect de l'invention, la membrane de biopolymère minéralisée est recouverte, sur ses deux faces, de nanocristaux d'apatite de formule générale Me₁₀(XO₄)₆Y₂, où Me représente un cation divalent, XO₄ un groupement anionique trivalent et Y un anion monovalent.

La structure apatitique accepte un grand nombre de substituants. Ainsi, les cations bivalents (Me²⁺) peuvent non seulement être remplacés par des cations bivalents (Mg²⁺, Ag²⁺, Zn²⁺, Sr²⁺, Ba²⁺, Pb²⁺), mais également par des cations monovalents (e.g. Na+, K+) ou trivalents (La³⁺, Ga³⁺, Eu³⁺). De même, les anions XO₄³⁻ trivalents peuvent être remplacés par des groupements bivalents (CO₃², SO4², HPO₄²⁻), trivalents (AsO₄³⁻, VO₄³⁻ ) ou tétravalents (SiO₄⁴⁻). Enfin, les groupements Y- peuvent également être substitués par des ions monovalent (F-, Cl-) ou bivalents (CO₃², O²⁻, S²⁻).

Toutes les combinaisons de cations et d'anions possibles peuvent être envisagées selon le procédé de l'invention, en fonction des solutions utilisées lors de la mise en oeuvre dudit procédé.

Le tableau 2 ci-dessous, extrait de la thèse d'Antoine BOYER intitulée « Synthèse, Caractérisation et Evaluation Biologique d'Apatites Phosphocalciques Carbo-Silicatées », présente différents types d'apatite pouvant être obtenues par le procédé selon l'invention, et présentant un intérêt biologique :

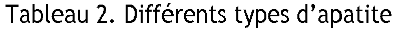

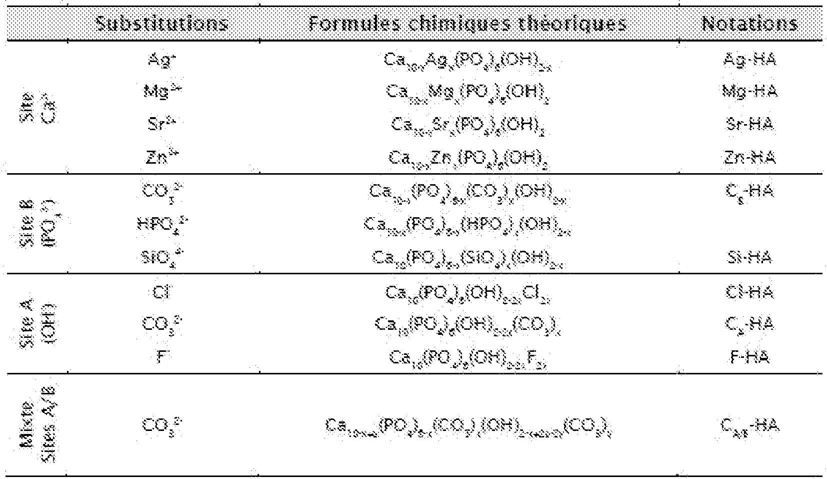

D'un point de vue biologique, le magnésium est intéressant en ce qu'il joue un rôle dans le remodelage osseux au moment de la calcification. L'argent a des propriétés antibactériennes. Le strontium a été retrouvé dans les tissus calcifiés de l'os et semble être un acteur important dans leur minéralisation. Le zinc a un effet inhibiteur sur l'activité ostéoclastique *in vitro.*

Pour ce qui est des substitutions anioniques, la présence d'ions fluorures F- est déterminante dans la croissance de l'os. L'absence de F- limite la densification de l'os, et inversement, sa trop forte concentration génère des ostéoscléroses. Les ions Cl- ont la capacité de développer un environnement acide à la surface de l'os, qui active les ostéoclastes dans le processus de résorption afin de solubiliser les sels alcalins du minéral osseux.

Ainsi, l'incorporation de tel ou tel ion dans la structure apatitique est susceptible de changer et potentiellement d'améliorer la bioactivité de la membrane de biopolymère minéralisée.

Selon un aspect particulier de l'invention, la membrane de biopolymère minéralisée est recouverte, sur ses deux faces, de nanocristaux d'hydroxyapatite de formule Ca₅(PO₄)₃(OH), souvent écrite Ca₁₀(PO₄)₆(OH)₂ pour souligner le fait que la maille de la structure cristalline comprend deux molécules.

Cette hydroxyapatite dite « stoechiométrique » contient 39% de Ca, 18,5% P et 3,38% de OH, les pourcentages étant exprimées en poids/poids total du minéral. L'hydroxyapatite est la principale composante minérale de l'émail dentaire, de la dentine et de l'os. Le numéro CAS de l'hydroxyapatite est 12167-74-7.

Selon un autre aspect particulier de l'invention, la membrane de biopolymère minéralisée est recouverte, sur ses deux faces, de nanocristaux de brushite (phosphate dicalcique hydraté, de formule CaHPO₄.2H₂O) et de nanocristaux d'hydroxyapatite carbonatée déficiente en calcium.

La membrane minéralisée selon l'invention présente de nombreux avantages et notamment une capacité de résorption très rapide pour une hydroxyapatite, après son introduction dans un organisme vivant.

Les membranes minéralisées selon l'invention peuvent avantageusement être totalement résorbées dans un temps compris entre 5 et 60 jours après leur implantation dans un organisme vivant. Une membrane selon l'invention pourra en particulier être résorbée en moins de 10 jours, en moins de 15 jours, en moins de 20 jours, en moins de 25 jours, en moins de 30 jours, en moins de 40 jours, en moins de 50 jours ou encore en moins de 60 jours après son implantation dans un organisme vivant, humain ou animal.

Ces temps de résorption ont été mesurés chez plusieurs modèles animaux (lapins, rats, chiens) comme cela est présenté dans l'exemple 6. On notera que dans la plupart des cas, la membrane ou la poudre est résorbée en moins de 25 jours ; toutefois pour certaines applications thérapeutiques particulières, comme par exemple la régénération de nerfs, il est nécessaire d'attendre jusqu'à 60 jours pour constater une résorption totale.

La membrane selon l'invention est parfaitement biocompatible, sans effet tumorigène, sans toxicité, sans effet immunigène, ce qui évite le rejet ou l'encapsulation de la membrane, grâce à une haute capacité de bio-intégration à l'organisme receveur.

La présente invention concerne également une membrane de biopolymère minéralisée sur ses deux faces avec des nanocristaux de composés minéraux organisés sous forme de plaquettes et de cristaux nanoporeux sphériques présentant une structure alvéolaire.

Ces composés minéraux pourront être l'un de ceux cités précédemment, notamment choisis parmi :
- Des composés phosphocalciques, notamment choisis parmi les suivants :
   ∘ De l'hydroxyapatite ; et/ou
   ∘ De l'hydroxyapatite déficiente dopée avec des ions fluorures, argent, ou zinc ; et/ou
   ∘ Une apatite poly-substituée ou une hydroxy, fluoro ou carboxy-apatite déficiente ; et/ou
   ∘ De la brushite (phosphate dicalcique hydraté : CaHPO₄.2H₂O) ; et/ou
   ∘ Des composés phosphocalciques avec des groupements hydrogéno-phosphate labiles,
- du sulfate de magnésium, ou encore
- du sulfate d'aluminium, ou
- toute combinaison de ces composés minéraux.

La présente invention concerne une membrane de biopolymère minéralisée sur ses deux faces avec des nanocristaux de phosphate de calcium organisés sous forme de plaquettes et de cristaux nanoporeux sphériques présentant une structure alvéolaire.

L'exemple 7 de la présente demande présente les caractéristiques structurelles de cette membrane, qui n'avaient jamais été observées jusqu'à présent. En particulier, l'organisation des nanocristaux sous forme de plaquettes et de cristaux nanoporeux sphériques présentant une structure alvéolaire n'est pas observée sur les échantillons témoins, qui présentent des agglomérats grossiers et des structures non homogènes. Cette structure des nanocristaux déposés sur la membrane selon l'invention est particulièrement avantageuse pour les raisons suivantes :
- les plaquettes ne contiennent pas de structures en « aiguilles » susceptibles de blesser les tissus de l'organisme dans lequel la membrane sera introduite ;
- les cristaux sphériques présentant une structure alvéolaire permettent de fixer des protéines biologiques circulantes autour de la membrane, après son implantation au sein d'un organisme vivant, lesdites protéines participant de manière active à la cicatrisation et à la résorption du matériel implanté.

Selon une mise en oeuvre particulière, le biopolymère constituant la membrane est du collagène.

La présente invention concerne également une membrane de biopolymère minéralisée sur ses deux faces avec des nanocristaux de composés minéraux, notamment de phosphate de calcium, organisés sous forme de plaquettes et de cristaux nanoporeux sphériques présentant une structure alvéolaire, susceptible d'être obtenue selon le procédé de l'invention, ou obtenue selon le procédé de l'invention.

D'autres objets de l'invention sont des formulations galéniques particulières de l'une de ces membranes de biopolymère minéralisées :
- une poudre constituée de l'une de ces membranes de biopolymère réduite en poudre, par un moyen choisi parmi ceux connus de l'Homme du métier, ou bien
- une solution comprenant ladite poudre en suspension dans un véhicule pharmaceutiquement acceptable.

Un véhicule pharmaceutiquement acceptable désigne des véhicules ou des excipients, c'est à dire des composés ne présentant pas d'action propre sur l'organisme. Ces véhicules ou excipients sont pharmaceutiquement acceptables, ce qui signifie qu'ils peuvent être administrés à un organisme vivant sans génération d'effets délétères significatifs.

Ainsi, selon l'invention, la poudre obtenue comprend un biopolymère, tel que défini précédemment, et des nanocristaux, notamment d'hydroxyapatite. En particulier, les nanocristaux seront organisés sous forme de plaquettes et de cristaux nanoporeux sphériques présentant une structure alvéolaire.

### Usages thérapeutiques

Lesdites membranes minéralisées pourront être introduites dans un organisme vivant, notamment d'un être humain, afin de réparer des désordres osseux, cartilagineux, ou d'autres organes de structure.

Une poudre de membrane minéralisée, ou une solution contenant celle-ci, pourront être administrées à un individu selon tout mode d'administration connu de l'Homme du métier, notamment par voie orale, sublinguale, par inhalation, sous-cutanée, intramusculaire, intraveineuse, transdermique, oculaire ou rectale.

Ainsi, la présente invention concerne une membrane de biopolymère, ou une poudre, ou une solution tels que décrits ci-dessus, pour son utilisation thérapeutique dans le traitement des désordres osseux, cartilagineux, pancréatiques, rénaux, urétraux, urétéraux et vésicaux, testiculaires, ovariens, intestinaux, hépatiques, neurologiques, cardiaques, tympaniques, oculaires, urinaires, gynécologiques, pulmonaires, bronchiques, trachéaux, vasculaires, conjonctifs, cutanés, muqueux, dentaires, gingivaux et/ou du tissu hématopoïétique et immunitaire.

En particulier, la présente invention concerne une membrane de biopolymère, ou une poudre, ou une solution tels que décrits ci-dessus, pour son utilisation thérapeutique dans le traitement des désordres pancréatiques.

En particulier, la présente invention concerne une membrane de biopolymère, ou une poudre, ou une solution tels que décrits ci-dessus, pour son utilisation thérapeutique dans le traitement des désordres rénaux.

En particulier, la présente invention concerne une membrane de biopolymère, ou une poudre, ou une solution tels que décrits ci-dessus, pour son utilisation thérapeutique dans le traitement des désordres urinaires.

En particulier, la présente invention concerne une membrane de biopolymère, ou une poudre, ou une solution tels que décrits ci-dessus, pour son utilisation thérapeutique dans le traitement des désordres dentaires.

En particulier, la présente invention concerne une membrane de biopolymère, ou une poudre, ou une solution tels que décrits ci-dessus, pour son utilisation thérapeutique dans le traitement des désordres gynécologiques.

En particulier, la présente invention concerne une membrane de biopolymère, ou une poudre, ou une solution tels que décrits ci-dessus, pour son utilisation thérapeutique dans le traitement des désordres hématopoïétiques et immunitaires.

En particulier, la présente invention concerne une membrane de biopolymère, ou une poudre, ou une solution tels que décrits ci-dessus, pour son utilisation thérapeutique dans le traitement de l'inflammation.

En particulier, la présente invention concerne une membrane de biopolymère, ou une poudre, ou une solution tels que décrits ci-dessus, pour son utilisation thérapeutique dans le traitement des désordres liés à une activité oxydante élevée.

L'invention se rapporte également à une méthode thérapeutique (non revendiquée) pour le traitement des désordres osseux, cartilagineux, pancréatiques, rénaux, urétraux, urétéraux et vésicaux, testiculaires, ovariens, intestinaux, hépatiques, neurologiques, cardiaques, tympaniques, oculaires, urinaires, gynécologiques, pulmonaires, bronchiques, trachéaux, vasculaires, conjonctifs, cutanés, muqueux, dentaires, gingivaux et/ou du tissu hématopoïétique et immunitaire chez un être humain, comprenant l'administration audit être humain d'une quantité efficace d'une membrane de biopolymère minéralisée, d'une poudre ou d'une solution tels que décrits dans la présente demande.

L'invention se rapporte également à un complément alimentaire, comprenant une poudre ou une solution telle que décrite ci-dessus.

La poudre ou solution selon l'invention n'agit qu'en cas d'inflammation, de blessure ou de maladie ; de plus, son activité est limitée dans le temps par les quantités disponibles. Cette poudre ou solution n'engendre pas de phénomène de réplication cellulaire anarchique ou incontrôlé. Elle n'a pas d'action sur la division cellulaire sans traumatisme préalable, et ce n'est pas un facteur de croissance.

La membrane de biopolymère minéralisée peut être utilisée en chirurgie, en tant que matériel « DMI » de classe 3 lors d'une intervention chirurgicale en intracorporel:
- Seul et sec ; ou
   - Associé à des antibiotiques (C3G, Vancomycine, gentamycine,...) ou des hormones pro-régénérantes (Ocytocine, sérotonine...).

Il peut être utilisé sous forme de « plaque » pour une implantation au contact des organes ou un dépôt en surface. Il n'est pas nécessaire d'utiliser une plaque de forme particulière, la régénération de l'organe s'organisant d'elle-même.

La régénération tissulaire débutera sur site dès la résorption du matériel dans les conditions physiologiques post-chirurgicales avec les médiateurs de l'inflammation et les cellules de l'hôte (macrophages M1 et M2, cellules différenciées et les cellules souches).
- Lorsque la membrane de biopolymère minéralisée est sous forme de poudre injectable (reconstituée dans du sérum physiologique ou de la xylocaïne), elle peut être utilisée :
   - en intra-osseux (pour un usage local ou général),
   - en intra articulaire,
   - dans les tissus mous (ex : dans la cloison nasale pour régénérer un défaut de la cloison) ou en mésothérapie,
   - en collyre ou en injection intra-vitréenne,
   - en goutte auriculaire ou nasale,
   - en solution capillaire ou cutanée,
   - dans un tube de collagène ou de chitosan résorbable reliant les extrémités neuronales, ou
   - en intravasculaire (stimule la néo angiogenèse à l'origine des phénomènes de régénération tissulaire).

La poudre peut également être administrée par pulvérisation :
- Bronchique et pulmonaire, ou
- Nasale (pour une voie cérébrale), ou encore
- Cutanée (poudre seule ou en préparation dans une crème spécifique).

### EXEMPLES

### Exemple 1. Procédé de minéralisation d'une membrane de collagène

Dans cet exemple, une membrane de collagène d'une taille de 100 cm² (10x10) est minéralisée sur ses deux faces avec des nanocristaux d'hydroxyapatite, selon le protocole décrit ci-après.

Le récipient utilisé est en verre. La fenêtre (ouverture de communication entre les deux compartiments) fait 10x10 cm.

### 1- Préparation des solutions

Deux solutions de chlorure de calcium et de phosphate d'ammonium sont préparées, par dissolution complète des poudres dans les béchers à l'aide de pipettes dédiées pour obtenir 2x 2.5 litres de :
- Phosphate d'ammonium (avec une, deux ou trois solutions de phosphate d'ammonium substituées (NH₄H₂PO₄, (NH₄)₂HPO₄, (NH₄)₃PO₄)), et
- Chlorure de calcium dans laquelle est rajoutée ultérieurement du Ca(OH)₂.

### 2- Préparation du « Sandwich » (assemblage (3))

Des feuilles de cellulose sont placées sur chaque face de la membrane de collagène.

### 3- Remplissage des bacs :

Il doit se faire à la même vitesse de chaque côté sans mélange des liquides, pour éviter que la pression du compartiment le plus rempli ne déforme l'assemblage.

Quelques gouttes d'une solution de NH₃ concentrée sont ajoutées dans le compartiment comprenant les ions PO₄³⁻.

Une solution d'hydroxyde de sodium est ajoutée dans le compartiment contenant les ions Ca⁺⁺.

### 4- Disposition des électrodes et de l'agitateur

L'électrode négative (cathode) est faite en acier, elle est plongée dans le bain de phosphate d'ammonium.

L'électrode positive (anode) est en graphite solide (carbone) ou en graphite (tissu de carbone), elle est plongée dans le bain de Ca⁺⁺ avec l'agitateur.

### 5- Démarrage du procédé

Un courant continu est appliqué aux électrodes de manière à obtenir une tension réelle comprise entre 2 Volts et 13 Volts.

Le procédé se déroule à température ambiante pendant 24 heures au total, pendant lesquelles les électrodes et les solutions sont interverties au bout de 12 heures.

### 6- Fin de procédure

Doucement, l'agitateur et l'électrophorèse sont arrêtés. Les électrodes sont retirées sans contaminer les solutions.

La membrane de collagène imbibée de nanocristaux d'hydroxyapatite est retirée et séchée à une température ne dépassant pas 30 - 35 ° C (presque à température ambiante) pendant 12 à 24 heures environ.

### Exemple 2. Effets constatés de l'administration des produits selon l'invention

- Des effets positifs de l'administration de ces produits selon l'invention ont été constatés dans les tissus suivants :
   - Osseux (traitement des pertes de substance et de pseudarthrose, traitement des ostéomyélites)
   - Cartilagineux (hyalin et articulaire) (régénération cartilagineuse)
   - Rénal (hémostase et renforcement de tranche de résection polaire rénale)
   - Hépatique (comblement de kystes hépatiques, hémostase et guide de la régénération hépatiques après hépatectomie)
   - Neurologique (régénération de diastasis sur des nerfs périphériques)
   - Cardiaque (revascularisation et régénération myocardique après infarctus)
   - Tympanique, oculaire (régénération de tympan, traitement du glaucome congénital)
   - Urinaire (ex : guide de cicatrisation d'hypospadias)
   - Gynécologique (traitement intravasculaire de la stérilité féminine, régénération du col utérin)
   - Pulmonaire (comblement de kyste ou de perte de substance, renforcement de suture bronchique)
   - Conjonctif et tissu de soutien (incontinence d'effort)
   - Cutané et muqueux (plaie, ulcère, gingivopathie ...), et
   - Dentaire (traitement des caries, greffe et création de dents, préparation osseuse avant geste d'implantologie).

### Exemple 3. Usage particulier en injection intra-médullaire osseuse : action à distance

La forme « poudre » peut être reconstituée sous forme d'une solution injectable (1ml de poudre sèche diluée par 2 à 4 ml de sérum physiologique et/ou xylocaïne 1% par exemple). Elle peut ensuite être injectée dans la moelle osseuse à l'aide d'une seringue et d'un trocart osseux, par exemple au niveau de la crête iliaque.

L'effet généré au niveau du tissu hématopoïétique et immunitaire va engendrer une diminution des réactions inflammatoires.

Par exemple, une amélioration et diminution de l'inflammation sur la tranche d'une hépatectomie peut être observée après une injection post-opératoire dans la crête iliaque.

L'administration de la poudre en suspension a une action positive au niveau d'une zone endommagée du myocarde, suite à une injection post-crise dans la crête iliaque.

### Exemple 4. Usage oral et muqueux buccal en tant que complément alimentaire

Les plaques et la poudre peuvent être administrées par voie buccale. L'absorption se fait à travers la muqueuse jugale et gingivale.

La prise se fait 2 fois par jour pendant 30 jours. L'effet constaté est :
- local avec une amélioration de la trophicité du tissu gingival, une tension retrouvée du ligament dentaire si les dents bougent ; et
- général avec une diminution des douleurs articulaires ou d'arthrose dès le 5ème jour de prise, une amélioration de l'humeur et de l'état général.

Chez les individus atteints de diabète de type 2, des effets positifs ont été observés sur l'équilibre glycémique après administration bi-journalière de la poudre selon l'invention.

### Exemple 5. Action anti-oxydante du produit après administration orale, mesurée dans les urines

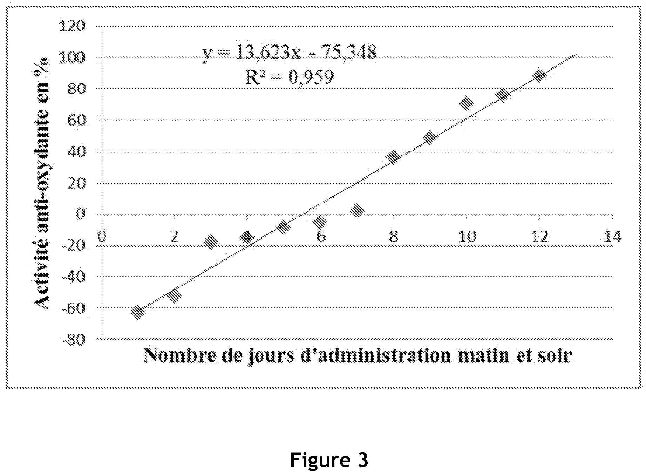

L'ingestion par des individus, matin et soir, d'une poudre selon la présente invention provoque une augmentation croissante de l'activité anti-oxydante totale mesurée sur des échantillons d'urine, comme cela est présenté en figure 3.

Le TAC (activité anti-oxydante totale) est un biomarqueur qui mesure le potentiel antioxydant des fluides corporels. L'Homme du métier connaît les différentes méthodes de test utilisées pour mesurer le TAC, et une bonne corrélation a été trouvée entre les résultats obtenus par différentes techniques. Les échantillons d'urine sont obtenus de manière non invasive, ce qui présente un intérêt considérable pour l'étude du statut antioxydant du corps.

Le protocole d'investigation est résumé ci-dessous :
3 participants en bonne santé générale ont été enrôlés dans l'étude, deux hommes âgés de 67 et 75 ans et une femme de 25 ans. La figure 3 présente les résultats obtenus chez l'homme âgé de 67 ans ; des résultats similaires ont été observés chez les deux autres individus testés.

Des échantillons témoins d'urine du matin ont été prélevés à 7 heures à jeun, puis à 8 heures et à 19 heures, selon le protocole usuel.

La détermination de l'activité anti-oxydante totale des échantillons a été réalisée par une méthode d'analyse coulométrique classique, mesurant la quantité d'électricité consommée ou produite pendant une électrolyse.

Les résultats sont présentés en pourcentages par rapport aux échantillons contrôle, le « standard » utilisé étant la rutine, un flavonoïde possédant des propriétés antioxydantes reconnues. Les échantillons d'urine ont été dosés avec des activités antioxydantes totales dont les valeurs en équivalence sont de 285 à 1647 mg de rutine (Ru) pour 1 dm³ (litre).

La figure 3 montre la nette augmentation de l'activité anti-oxydante totale des urines prélevées chez un patient consommant deux fois par jour une poudre selon l'invention, entre les premiers jours (jusqu'au 6^{ème} jour, l'activité mesurée est « oxydante » et non anti-oxydante), et les jours 10-12 où le pourcentage d'activité anti-oxydante des échantillons d'urine est élevé.

### Exemple 6. Résorption de la membrane obtenue par le procédé selon l'invention, dans différents modèles animaux (rat, lapin, chien)

La résorption de la membrane est déterminée par analyse histologique après implantation sur l'animal (rat, lapin ou chien) dans un tissu particulier, puis après biopsie de la zone implantée à différent temps.

La résorption constatée est rapide, la disparition progressive du matériau implanté fait place progressivement à des petits vaisseaux sanguins (néo-angiogenèse), du tissu conjonctif puis du tissu fonctionnel cellularisé.

L'étude spécifique des temps de résorption de la membrane minéralisées, sous forme de plaque ou de poudre, effectuée dans le tissu sous-cutané de chiens et de lapins, a montré que:
- A J2-J3 post-implantation (pi) : une résorption partielle de la membrane minéralisée, avec présence d'une grande proportion de granulocytes éosinophiles. Les fibres de collagènes sont gonflées.
- A J5-J7 pi : une résorption importante de la membrane minéralisée, avec présence de cellules géantes multi-nucléées, de lymphocytes et de macrophages.
- A J14 pi : une forte proportion de cellules mononucléaires au sein des quelques ilots de membrane minéralisée restants. On trouve, en lieu et place du composite résorbé, un tissu de granulation jeune et mature avec des vaisseaux formés par des endothéliocytes aplatis typiques remplis de cellules sanguines. On note également la transformation des granulations en tissu fibreux.
- A J25 pi : à la place de la membrane qui a disparue, on retrouve des îlots isolés de tissu conjonctif mature en plein développement entourés de macrophages, de cellules géantes, et de lymphocytes. La membrane n'a pas provoqué de réaction de rejet. Les changements morphologiques dans la zone d'implantation de la membrane correspondent à une forte réaction cellulaire proliférative avec remplacement du complexe collagène-apatite par du tissu conjonctif fibreux.

Les tableaux 3 à 8 ci-dessous présentent l'évolution histologique constatée en fonction du tissu hôte et du délai après implantation (pi : post implantation).

**Tableau 3. Implantation d'une membrane minéralisée pour reconstruction d'un os (chien, n=16)**

| 3^{ème} jour pi | 14^{ème} jour pi | 25^{ème} jour pi |
|---|---|---|
| Le matériel implanté est résorbé partiellement mais encore présent en | Le matériel est résorbé massivement. Il est entouré de macrophages et | On ne retrouve pas de matériel implanté. Le tissu formé présente des |
| majorité. Il se constitue un tissu conjonctif lâche en périphérie. | de nombreuses cellules géantes à corps étranger. | vaisseaux sanguins, des ostéoclastes, des ostéoblastes et des ostéocytes. |

**Tableau 4. Implantation d'une membrane minéralisée dans le poumon et la plèvre (lapin, n=8)**

| 3^{ème} jour pi | 7^{ème} jour pi | 14^{ème} jour pi | | 25^{ème} jour pi |
|---|---|---|---|---|
| Biodégradation rapide et partielle du matériel, présence de polynucléaires éosinophiles dans des fibres de collagène amincies et lysées | Résorption complète du matériel implanté, formation de « précapillaires » en lieu et place (ébauche de capillaires sanguins) formés par des réticulocytes et des cellules mésenchymateuses | Ebauche de capillaires sanguins avec | | Capillaires sanguins contenant des cellules sanguines circulantes, entourés de tissus conjonctif et de parenchyme pulmonaire |
| | | | - Une paroi vasoïde, | |
| | | | - Des cellules faiblement différenciées avec formation de la rosette érythrocytaire à l'intérieur | |
| | | | - Des Cellules mésenchymateuses entrant en mitose autour des parois | |
| | | | - Du parenchyme pulmonaire entourant le tissu conjonctif formé | |
| | | Accumulation de nombreux polynucléaires neutrophiles et éosinophiles | | |

**Tableau 5. Foie - implantation d'une membrane minéralisée dans une cavité kystique créée (rats, n=33)**

| 7^{ème} jour pi | 14^{ème} jour pi | 25^{ème} jour pi |
|---|---|---|
| Apparition d'un tissu de granulation, diminution des exsudats | Poursuite du processus de formation du tissu de granulation à la place du composite | Maturation de ce tissu de granulation avec présence d'un tissu conjonctif avec des structures organotypiques : des hépatocytes et des cholangioles avec des signes de recanalisation régionale des voies biliaires. |
| | | Les capillaires sinusoïdaux et biliaires ont conservé leur lumière et ne présentaient aucun signe de perturbation de la microcirculation sanguine ni de l'excrétion de bile. |
| | | Hypertrophie des hépatocytes adjacents avec augmentation de leur activité mitotique (multipliée par 3) et augmentation de la synthèse de la protéine Ki-67 (multipliée par 3 également). |

**Tableau 6. Injection dans la moelle osseuse (crête iliaque) d'une poudre constituée de membrane minéralisée broyée, et hépatectomie (chien, n=5)**

| 14^{ème} jour pi | 25^{ème} jour pi | 45^{ème} jour pi |
|---|---|---|
| Disparition complète du matériel implanté. | Nombreuses cellules souches mésenchymateuses pluripotentes dans la moelle osseuse mais également dans la circulation générale et au niveau de la tranche d'hépatectomie (nombreuses cellules souches en mitose) | Au sein de la tranche d'hépatectomie, phénomène de régénération hépatique avec apparition de canaux biliaires interlobaires nouvellement formés |
| Entre les trabécules osseuses, on retrouve de nombreuses cellules souches mésenchymateuses pluripotentes | | |

**Tableau 7. Injection dans la moelle osseuse (crête iliaque) d'une poudre constituée de membrane minéralisée broyée, et lien avec maladie dégénérative cérébrale par perte de microvascularisation, perte des neurones et prolifération des cellules gliales. (chien, n=1)**

| 7^{ème} jour pi | 14^{ème} jour pi | 45^{ème} jour pi |
|---|---|---|
| Dans la moelle : résorption du matériel, présence de cellules souches mésenchymateuses pluripotentes et de fibres de collagène (structure extracellulaire). | Au niveau cérébral : Une biopsie du cortex cérébral (gyrus) met en évidence des modifications mineures des neurocytes au milieu d'un manque de cellules gliales | Au niveau de la moelle osseuse : capillaires en cours de croissance avec des globules rouges dans la lumière, Cluster de cellules souches mésenchymateuses pluripotentes. |
| | | Dans le cortex cérébral : |
| | | Activation de l'angiogenèse (Capillaire nouvellement formé. Mitoses endothéliocytaires). |
| | | Réduction du nombre de cellules gliales et restauration des neurocytes |

Tableau 8. Traitement de la coupure d'un nerf périphérique chez le rat (rat, n=5).

Une perte de substance nerveuse de 1 cm était observée sur le nerf sciatique. Une solution composée de poudre issue du broyage d'une membrane minéralisée a été placée dans un tube de kératine qui joint les 2 extrémités du nerf rompu.

Les observations cliniques sont consignées dans le tableau 8 ci-dessous.

| 14^{ème} jour pi | 25^{ème} jour pi | 60^{ème} jour pi | 90^{ème} jour pi | |
|---|---|---|---|---|
| Biodégradation séparée de l'implant et formation de la régénération cellulaire neuronale. Formation d'un lit microvasculaire. | Fibres de myéline en formation sous forme de cylindres axiaux. Ce processus est organisé par des neurolemmocytes | La poudre | Présence de : | |
| | | injectée est complètement biodégradée et à la place du diastasis, un fragment du nerf sciatique s'est formé, composé de fibres amyéliniques (≈ 71% de leur nombre dans le nerf sciatique intact) et de myéline (≈ 68% de leur nombre dans le nerf sciatique intact). Les faisceaux nerveux sont entourés de tissu conjonctif. | | - Microvaisseaux dans le fragment restauré du nerf sciatique opéré : avec des artérioles, des veinules (contenant des érythrocytes et des endotélocytes) |
| | | | | - Tissu conjonctif |
| | | | | - Des fibres nerveuses myélinisées |

### Exemple 7. Caractérisation moléculaire des cristaux de phosphate de calcium présents sur les membranes obtenues par le procédé selon l'invention

Les nanocristaux de phosphate de calcium obtenus par plusieurs procédés différents de minéralisation ont été comparés :
- Echantillon 7 : Membrane de collagène minéralisée pendant 24 heures, Sans Cellophane - Sans Electrophorèse
- Echantillon 4 : Membrane de collagène minéralisée pendant 24 heures, Avec Cellophane - Sans Electrophorèse
- Echantillon 6 : Membrane de collagène minéralisée pendant 24 heures, Sans Cellophane - Avec Electrophorèse
- Echantillon 5 : Membrane de collagène minéralisée pendant 24 heures, Avec Cellophane - Avec Electrophorèse, soit selon le procédé de l'invention.

Les échantillons ont été analysés avec les techniques de caractérisations suivantes :
- Microscope électronique à balayage (MEB) Zeiss Supra 55VP. Observations réalisées sans dépôt de surface conducteur et à basse tension pour ne pas masquer la morphologie des agglomérats et cristaux de phosphates de calcium formés à la surface des fibres de collagène.
- Diffractomètre à rayons X (DRX) Bruker D8 Advance 9/9 XRD équipé d'un détecteur Lynx-Eye Position Sensitive (angle ouverture 2.946 °), utiliasnt la radiation CuK α (40 kV and 40 mA). Les diffractogramme ont été analysés à l'aide du logiciel TOPAS-64 V6.
- Spectromètre à infrarouge (IR) VERTEX 70 (Bruker Optics, France), équipé d'un système à réflectance totale atténuée (Quest ATR diamond; Specac, USA). Afin de pouvoir comparer les spectres les uns avec les autres une procédure de « curve fitting » avec ajout d'un étalon a été réalisée, selon la procédure développée dans la thèse de Baptiste Charbonnier (EMSE, 09/12/2016).

### A- Analyse par microscopie électronique

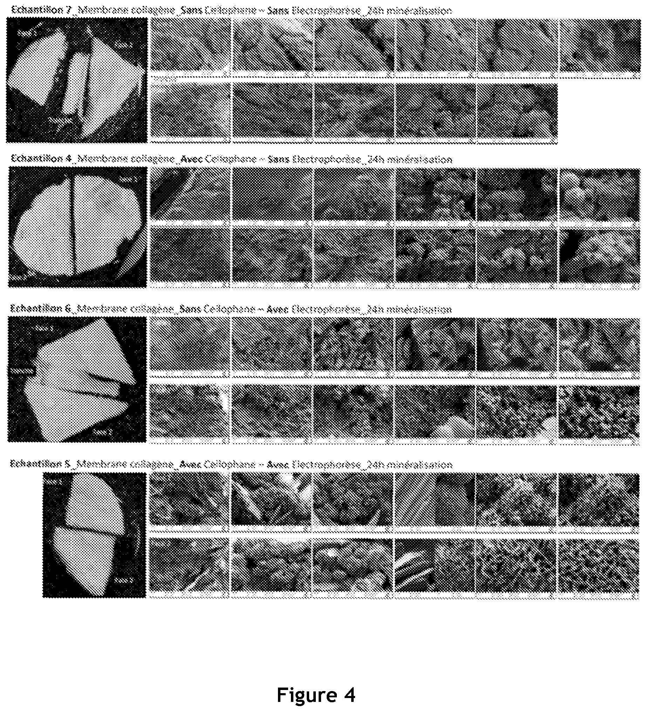

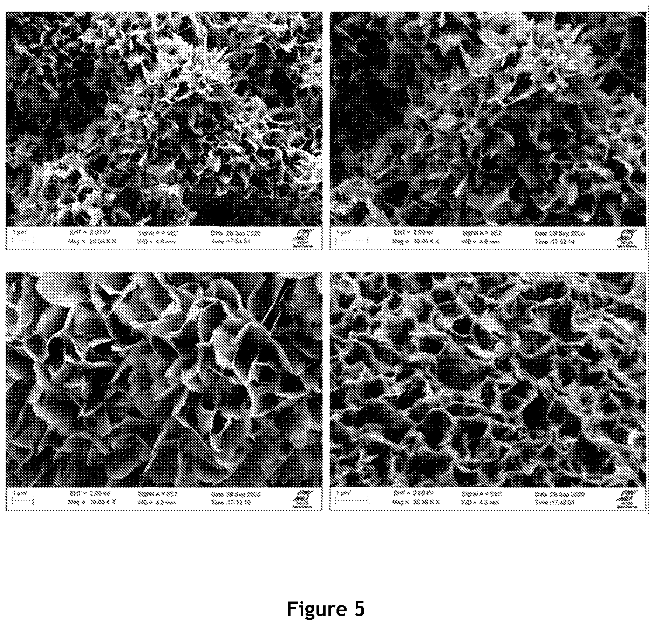

Les figures 4 et 5 présentent des images obtenues par microscopie électronique à balayage (MEB) sur les deux faces des membranes de collagènes minéralisées, à plusieurs grossissements.

Les images montrent que :
- l**'échantillon 7** obtenu **sans** une membrane poreuse de **cellophane** et **sans électrophorèse** est très épaisse, très minéralisé et assez dense, voire obstruée/colmatée pour la Face 1 (grossissement ≥ x10.000). Macroscopiquement la matrice minéralisée 7 ressemble à une bande de plâtre. La morphologie des agglomérats de surface est grossière des deux côtés de la membrane à faible grossissement. Une relative inhomogénéité de la morphologie des cristaux de CaP apparaît à plus fort grossissement (≥ x10.000) entre les 2 faces de la membrane.
- **l'échantillon 4** obtenu **avec** une membrane poreuse de **cellophane** et **sans électrophorèse** est contrairement à l'échantillon 7 très fin et très peu minéralisé. La Face 1 présente nettement la matrice de collagène (couleur jaune sur la photo). La couche de minéral à cette surface est très fine mais très dense ; la porosité semble totalement fermée/obstruée. Des petites boules de minéral ≤ 1 µm en diamètre sont visibles aléatoirement sur cette Face (1) alors qu'elles ne le sont pas sur l'autre Face (2). La différence entre la Face 1 et la Face 2 est évidente.
- l'échantillon 6 obtenu sans membrane poreuse de cellophane et avec électrophorèse est comme l'échantillon 7 relativement épais, avec toutefois des variations nettes d'épaisseur (simple au double, photo Echantillon 6). Le minéral formé de part et d'autre de la membrane de collagène (i.e., Faces 1 et 2) est relativement plus poreux que pour l'échantillon 7 avec d'un côté (Face 1) des agglomérats grossiers et aléatoirement dispersés (environ 10 µm diamètre) et une nanostructure inhomogène, et de l'autre côté (Face 2) une surface composée de petites boules de minéral ≤ 1 µm en diamètre. La concentration de ces boules est nettement plus élevée que pour la Face 1 de l'échantillon 4.
- l**'échantillon 5** obtenu **avec** une membrane poreuse de **cellophane** et **avec électrophorèse** est significativement différent des 3 autres que ce soit à l'échelle de la membrane, à l'échelle des agglomérats ou à l'échelle nanométrique.

Macroscopiquement la membrane a le même aspect sur les 2 faces (photo, Echantillon 5). L'épaisseur et la couleur de minéral est relativement homogène sur les 2 faces avec des petits cristaux brillants.

Ces cristaux visibles sur les images MEB sont des plaquettes/feuillets de phosphates de calcium assez caractéristiques de la phase Brushite (CaHPO4, 2H2O), « leaflet shape » en anglais. Ces plaquettes sont relativement grandes jusqu'à plus de 100 µm de long et fine < 1 µm d'épaisseur (croissance selon un plan cristallographique bien précis). Les surfaces sont relativement poreuses avec des topographies accidentées.

Cette topographie est le fruit de l'association de cristaux sous forme de plaquette et de cristaux nanoporeux sphériques. Ces derniers ont un diamètre d'une dizaine de µm et possède des submicropores ressemblant aux cellules d'une ruche. Les parois de ces cellules, i.e., le cristal, sont très très fine, de l'ordre du nanomètre.

La figure 5 présente les images obtenues par microscopie électronique à balayage sur les deux faces de la membrane, aux grossissements x20.000 et x30.000.

Cette morphologie des nanocristaux est très particulière et très intéressante. En effet, la taille et la forme de la porosité peut piéger des protéines biologiques d'intérêt (facteurs de croissance ou de cicatrisation, tels que BMP2, etc). Les parois fines en forme de cellules de ruche (alvéoles) augmentent considérablement la surface d'échange avec les fluides et ainsi la capacité d'interaction de ces nanocristaux avec ces derniers.

En conclusion, le procédé de minéralisation de membrane de collagène selon l'invention, associant une membrane poreuse (type cellophane) et un champ électrique, forme effectivement des morphologies de cristaux uniques et orientées, ainsi qu'une répartition homogène de ces cristaux de part et d'autre de la membrane.

### B- Diffractogrammes des membranes minéralisées broyées manuellement

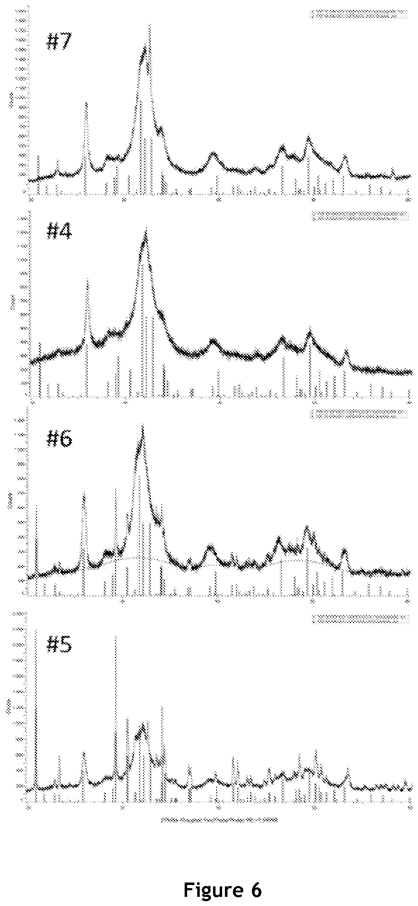

La figure 6 représente une comparaison des spectres de diffraction obtenus sur les membranes des échantillons 7, 4, 6 et 5 broyés.

Tous les échantillons présentent des spectres avec des pics de diffraction plutôt larges et peu intenses.

Les échantillons **7** et **4,** obtenus **sans électrophorèse,** présentent une seule et unique phase cristalline détectable dont la structure cristallographique est proche de celle de l'hydroxyapatite (indexée avec la fiche PDF 09-0432).

Les échantillons **6** et **5,** obtenus **avec électrophorèse,** présentent deux phases cristallines dont la structure cristallographique est pour l'une proche de celle de l'hydroxyapatite (HA, PDF 09-0432) et pour l'autre clairement celle de la Brushite (PDF 09-0077). Les pics de diffraction relatif à la Brushite sont également plus fins que ceux de la structure apatitique indiquant que l'ordre local des cristaux de Brushite est plus élevé que celui des cristaux d'apatite.

Ces résultats associés aux précédents confirment que les plaquettes observées au MEB sur l'échantillon 5 (procédé selon l'invention) sont des cristaux de Brushite plutôt bien cristallisés avec une croissance selon un plan cristallographique bien précis, et que les boules submicroporeuses sont des cristaux de structure cristallographique proche de celle de l'hydroxyapatite (HA).

### C- Spectres à infrarouge (IR) obtenus sur les membranes minéralisées broyées manuellement, normalisés

Les résultats confirment que l'échantillon 4, élaboré avec une membrane poreuse de cellophane et sans électrophorèse, est moins minéralisé que les autres échantillons.

Les échantillons 7 et 6 obtenus sans membrane de cellophane présentent des spectres IR équivalents avec les bandes caractéristiques des groupements phosphates (1200-900 cm⁻¹, 620-480 cm⁻¹), des groupements hydroxydes (3500 cm⁻¹, 620 cm⁻¹) et carbonates (1700-1200 cm⁻¹, 910-830 cm⁻¹). Ces échantillons sont donc composés essentiellement d'hydroxyapatites déficientes carbonatées de formule générale :
Ca10-x (PO4)6-x.(HPO4²⁻ ou CO3²⁻)x.(OH ou 1/2CO3)2-x avec 0 ≤ x ≤ 2

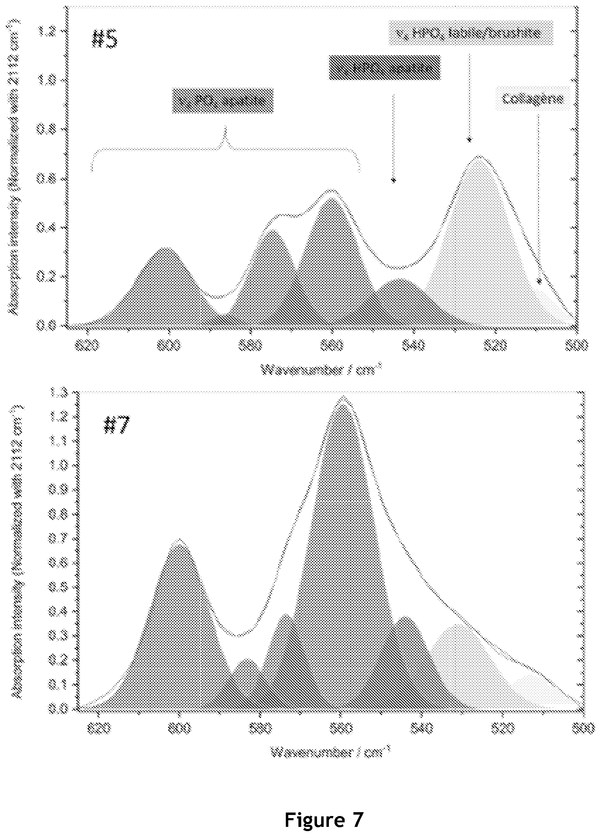

Ces échantillons présentent également une bande à 542 cm⁻¹ relative aux groupements HPO4 labile (Cf. Figure 7, graphe du bas). Cette bande est attribuable soit à des ions HPO4 piégés en dehors de la structure apatitique ou dans une Brushite.

De façon équivalente l'échantillon 5, élaboré avec une membrane poreuse de cellophane et sous électrophorèse, présente des bandes relatives aux groupements apatitiques ainsi qu'une bande relative aux groupements HPO4 labile (Cf. Figure 7, graphe du haut). Toutefois, les bandes relatives à l'apatite sont moins intense que celles observées sur le spectre de l'échantillon 7, alors que la bande à 542 cm⁻¹, relative aux groupements HPO4 labile, est plus intense. Ces résultats semi-quantitatif confirment les observations MEB et DRX précédentes.

En outre, l'échantillon 7 semble être plus minéralisé que l'échantillon 5 (Figure 7, bande phosphate à 560 cm⁻¹ / bande collagène à 510 cm⁻¹). Ce résultat confirme également les observations visuelles faites au MEB et l'aspect dense et obstrué/colmaté des surfaces des échantillons obtenus sans électrophorèse.

En conclusion, le procédé selon l'invention mettant en oeuvre une minéralisation contrôlée de membrane poreuse organique **à l'aide d'une** membrane **poreuse** de **cellophane** et **d'un champ électrique** permet effectivement d'obtenir une densité minérale relativement homogène au sein de la membrane organique, ainsi qu'une morphologie des cristaux de CaP et une composition du minéral unique. Les caractéristiques morphologiques des cristaux d'hydroxyapatite carbonatée déficiente en calcium, très orientés et en forme de cellule de ruche, sont particulièrement intéressants pour des applications biologiques (e.g., piégeage des protéines dans la submicroporosité, rapport surface d'échange/masse de cristal très élevé).

### REFERENCES BIBLIOGRAPHIQUES

EP 3181158
WO 2008/096334
RU2410040
WO 2013/111077
WO 2013/190534
US 6,395,036
WO 2008/096334
EP 3021883
RU2174848
US 5,532,217
US 5,739,286
US 6,589,590

Thèse d'Antoine BOYER intitulée « SYNTHESE, CARACTERISATION ET EVALUATION BIOLOGIQUE D'APATITES PHOSPHOCALCIQUES CARBO-SILICATEES », soutenue à Saint-Etienne le 17 avril 2014

## Revendications

1. Procédé de minéralisation d'une membrane de biopolymère, comprenant les étapes suivantes :
a) Introduction d'un assemblage (3) constitué d'une membrane de biopolymère (4) comprise entre deux feuilles de cellulose (A) et (B), dans un récipient comprenant :
- un premier compartiment (1) et un second compartiment (2), comprenant chacun une électrode, une première électrode étant une anode placée dans le premier compartiment (1) et une seconde électrode étant une cathode placée dans le second compartiment (2),
- les parois du premier compartiment (1) et du second compartiment (2) mises en contact l'une avec l'autre présentant chacune une ouverture mettant en communication le premier et le second compartiment,
l'assemblage (3) étant disposé dans ladite ouverture entre le premier et le second compartiment de manière à l'obturer, la feuille (A) de cellulose étant du côté du premier compartiment (1) et la feuille (B) de cellulose du côté du deuxième compartiment (2),
b) remplissage du premier compartiment (1) avec une solution aqueuse contenant au moins un cation choisi parmi : des ions calcium, des ions argent, des ions zinc, des ions cuivres, des ions sodium, des ions magnésium et des ions aluminium, et du second compartiment (2) avec une solution aqueuse contenant au moins un anion choisi parmi des ions fluorure, des ions sulfates, des ions carbonates, des ions silicates et des ions phosphate ;
c) Application d'une tension électrique entre les électrodes ;
d) retournement de l'assemblage (3) de manière à ce que la feuille (A) de cellulose soit du côté du second compartiment et la feuille (B) de cellulose du côté du premier compartiment, ou échange des solutions et électrodes du premier et du second compartiment;
c') application d'une tension électrique entre les électrodes, ladite tension étant égale à celle appliquée à l'étape (c) et étant appliquée pendant une durée identique à celle de l'étape (c) ;
e) retrait et rinçage de l'assemblage (3) ;
f) récupération et séchage de la membrane de biopolymère minéralisée.

2. Procédé selon la revendication 1, **caractérisé en ce que** la membrane de biopolymère est une membrane de collagène, d'alginate ou de chitosan.

3. Procédé selon l'une des revendications 1 à 2, **caractérisé en ce que** la solution aqueuse mise dans le premier compartiment (1) contient au moins des ions calcium, et la solution aqueuse mise dans le second compartiment (2) contient au moins des ions phosphate.

4. Procédé selon l'une des revendications 1 à 3, **caractérisé en ce que** lors des étapes (c) et (c'), la tension électrique appliquée est de 2 à 13 Volts.

5. Procédé selon l'une des revendications 1 à 4, **caractérisé en ce que** l'étape (c) d'application d'une tension électrique dure de 8 à 16 heures.

6. Procédé selon l'une des revendications 1 à 5, **caractérisé en ce que** lors des étapes (c) et (c'), la solution aqueuse contenant au moins un cation est agitée en continu.

7. Procédé selon l'une des revendications 1 à 6, **caractérisé en ce que** lors des étapes (c) et (c'), la solution aqueuse contenant au moins un cation est saturée en cations par un ajout continu d'hydroxyde dudit cation.

8. Procédé selon l'une des revendications 1 à 7, **caractérisé en ce que** le pH de la solution aqueuse contenant au moins un anion est compris entre 7 et 11.

9. Procédé selon l'une des revendications 1 à 8, **caractérisé en ce que** l'étape (f) de séchage de la membrane de biopolymère minéralisée est effectuée à une température inférieure à 35°C, de préférence pendant 12 à 24 heures.

10. Membrane de biopolymère minéralisée sur ses deux faces avec des nanocristaux de composés minéraux organisés sous forme de plaquettes et de cristaux nanoporeux sphériques présentant une structure alvéolaire, susceptible d'être obtenue par le procédé selon l'une des revendications 1 à 9.

11. Membrane selon la revendication 10, **caractérisée en ce que** le biopolymère est du collagène.

12. Membrane selon l'une des revendications 10 à 11, **caractérisée en ce que** les nanocristaux sont constitués de phosphate de calcium.

13. Poudre constituée d'une membrane de biopolymère minéralisée selon l'une des revendications 10 à 12, réduite en poudre.

14. Solution comprenant une poudre selon la revendication 13 en suspension dans un véhicule pharmaceutiquement acceptable.

15. Membrane de biopolymère minéralisée selon l'une des revendications 10 à 12, ou poudre selon la revendication 13, ou solution selon la revendication 14, pour son utilisation thérapeutique dans le traitement des désordres osseux, cartilagineux, pancréatiques, rénaux, urétraux, urétéraux et vésicaux, testiculaires, ovariens, intestinaux, hépatiques, neurologiques, cardiaques, tympaniques, oculaires, urinaires, gynécologiques, pulmonaires, bronchiques, trachéaux, vasculaires, conjonctifs, cutanés, muqueux, dentaires, gingivaux et/ou du tissu hématopoïétique et immunitaire.

## Patentansprüche

1. Verfahren zur Mineralisierung einer Biopolymermembran, umfassend die folgenden Schritte:
a) Einbringen einer Anordnung (3), die aus einer Biopolymermembran (4) zwischen zwei Zelluloseblättern (A) und (B) besteht, in einen Behälter umfassend:
- eine erste Kammer (1) und eine zweite Kammer (2), die jeweils eine Elektrode umfassen, wobei eine erste Elektrode eine Anode ist, die in der ersten Kammer (1) angeordnet ist, und eine zweite Elektrode eine Kathode ist, die in der zweiten Kammer (2) angeordnet ist,
- die Wände der ersten Kammer (1) und der zweiten Kammer (2), die miteinander in Kontakt stehen und jeweils eine Öffnung aufweisen, die die erste und die zweite Kammer miteinander verbindet,
wobei die Anordnung (3) in der Öffnung zwischen der ersten und der zweiten Kammer so angeordnet ist, dass sie diese verschließt, wobei sich das Zelluloseblatt (A) auf der Seite der ersten Kammer (1) und das Zelluloseblatt (B) auf der Seite der zweiten Kammer (2) befindet,
b) Befüllen der ersten Kammer (1) mit einer wässrigen Lösung, die mindestens ein Kation enthält, ausgewählt aus: Calciumionen, Silberionen, Zinkionen, Kupferionen, Natriumionen, Magnesiumionen und Aluminiumionen, und der zweiten Kammer (2) mit einer wässrigen Lösung, die mindestens ein Anion enthält, ausgewählt aus Fluoridionen, Sulfationen, Carbonationen, Silikationen und Phosphationen;
c) Anlegen einer elektrischen Spannung zwischen den Elektroden;
d) Wenden der Anordnung (3), so dass sich das Zelluloseblatt (A) auf der Seite der zweiten Kammer und das Zelluloseblatt (B) auf der Seite der ersten Kammer befindet, oder Austauschen der Lösungen und Elektroden der ersten und der zweiten Kammer;
c') Anlegen einer elektrischen Spannung zwischen den Elektroden, wobei die Spannung gleich der in Schritt (c) angelegten Spannung ist und für die gleiche Zeit wie in Schritt (c) angelegt wird;
e) Herausnehmen und Spülen der Anordnung (3);
f) Rückgewinnen und Trocknen der mineralisierten Biopolymermembran.

2. Verfahren nach Anspruch 1, **dadurch gekennzeichnet, dass** die Biopolymermembran eine Kollagen-, Alginat- oder Chitosanmembran ist.

3. Verfahren nach einem der Ansprüche 1 bis 2, **dadurch gekennzeichnet, dass** die in die erste Kammer (1) eingebrachte wässrige Lösung mindestens Calciumionen enthält und die in die zweite Kammer (2) eingebrachte wässrige Lösung mindestens Phosphationen enthält.

4. Verfahren nach einem der Ansprüche 1 bis 3, **dadurch gekennzeichnet, dass** in den Schritten (c) und (c') die angelegte elektrische Spannung 2 bis 13 Volt beträgt.

5. Verfahren nach einem der Ansprüche 1 bis 4, **dadurch gekennzeichnet, dass** der Schritt (c) des Anlegens einer elektrischen Spannung 8 bis 16 Stunden dauert.

6. Verfahren nach einem der Ansprüche 1 bis 5, **dadurch gekennzeichnet, dass** in den Schritten (c) und (c') die wässrige Lösung, die mindestens ein Kation enthält, kontinuierlich bewegt wird.

7. Verfahren nach einem der Ansprüche 1 bis 6, **dadurch gekennzeichnet, dass** in den Schritten (c) und (c') die wässrige Lösung, die mindestens ein Kation enthält, durch kontinuierliche Zugabe von Hydroxid dieses Kations mit Kationen gesättigt ist.

8. Verfahren nach einem der Ansprüche 1 bis 7, **dadurch gekennzeichnet, dass** der pH-Wert der wässrigen Lösung, die mindestens ein Anion enthält, zwischen 7 und 11 beträgt.

9. Verfahren nach einem der Ansprüche 1 bis 8, **dadurch gekennzeichnet, dass** der Schritt (f) des Trocknens der mineralisierten Biopolymermembran bei einer Temperatur unter 35°C, vorzugsweise 12 bis 24 Stunden lang, durchgeführt wird.

10. Biopolymermembran, die auf beiden Seiten mit Nanokristallen aus mineralischen Verbindungen mineralisiert ist, die in Form von Plättchen und kugelförmigen nanoporösen Kristallen mit einer wabenförmigen Struktur organisiert sind, und durch das Verfahren nach einem der Ansprüche 1 bis 9 erzielt werden kann.

11. Membran nach Anspruch 10, **dadurch gekennzeichnet, dass** das Biopolymer Kollagen ist.

12. Membran nach einem der Ansprüche 10 bis 11, **dadurch gekennzeichnet, dass** die Nanokristalle aus Calciumphosphat bestehen.

13. Pulver bestehend aus einer zu Pulver zermahlenen mineralisierten Biopolymermembran nach einem der Ansprüche 10 bis 12.

14. Lösung umfassend ein Pulver nach Anspruch 13, das in einem pharmazeutisch unbedenklichen Träger suspendiert ist.

15. Mineralisierte Biopolymermembran nach einem der Ansprüche 10 bis 12 oder Pulver nach Anspruch 13 oder Lösung nach Anspruch 14 zur therapeutischen Verwendung bei der Behandlung von Erkrankungen im Zusammenhang mit Knochen, Knorpeln, der Bauchspeicheldrüse, den Nieren, der Harnröhre, dem Harnleiter und der Blase, den Hoden, den Eierstöcken, dem Darm, der Leber, neurologischen Erkrankungen, Erkrankungen im Zusammenhang mit dem Herzen, dem Trommelfell, den Augen, den Harnwegen, gynäkologischen Erkrankungen, Erkrankungen im Zusammenhang mit der Lunge, den Bronchen, der Luftröhre, den Gefäßen, der Bindehaut, der Haut, der Schleimhaut, den Zähnen, dem Zahnfleisch und/oder dem hämatopoetischen Gewebe und dem Immunsystem.

## Claims

1. A method for mineralizing a biopolymer membrane, comprising the following steps:
a) Introducing an assembly (3) consisting of a biopolymer membrane (4) comprised between two cellulose sheets (A) and (B), into a container comprising:
- a first compartment (1) and a second compartment (2), each comprising an electrode, a first electrode being an anode placed in the first compartment (1) and a second electrode being a cathode placed in the second compartment (2),
- the walls of the first compartment (1) and of the second compartment (2) in contact with each other having each an opening putting into communication the first and the second compartment,
the assembly (3) being disposed in said opening between the first and the second compartment so as to close it, the cellulose sheet (A) being on the side of the first compartment (1) and the cellulose sheet (B) on the side of the second compartment (2),
b) filling the first compartment (1) with an aqueous solution containing at least one cation selected from: calcium ions, silver ions, zinc ions, copper ions, sodium ions, magnesium ions and aluminum ions, and the second compartment (2) with an aqueous solution containing at least one anion selected from fluoride ions, sulphate ions, carbonate ions, silicate ions and phosphate ions;
c) applying an electric voltage between the electrodes;
d) turning the assembly (3) over so that the cellulose sheet (A) is on the side of the second compartment and the cellulose sheet (B) on the side of the first compartment, or exchanging the solutions and electrodes of the first and the second compartment;
c') applying an electric voltage between the electrodes, said voltage being equal to that applied in step (c) and being applied for a duration identical to that of step (c);
e) removing and rinsing the assembly (3);
f) recovering and drying the mineralized biopolymer membrane.

2. The method according to claim 1, **characterized in that** the biopolymer membrane is a membrane of collagen, alginate or chitosan.

3. The method according to one of claims 1 to 2, **characterized in that** the aqueous solution placed in the first compartment (1) contains at least calcium ions, and the aqueous solution placed in the second compartment (2) contains at least phosphate ions.

4. The method according to one of claims 1 to 3, **characterized in that** during steps (c) and (c'), the electrical voltage applied is of 2 to 13 volts.

5. The method according to one of claims 1 to 4, **characterized in that** step (c) of applying an electric voltage lasts from 8 to 16 hours.

6. The method according to one of claims 1 to 5, **characterized in that** during steps (c) and (c'), the aqueous solution containing at least one cation is stirred continuously.

7. The method according to one of claims 1 to 6, **characterized in that** during steps (c) and (c'), the aqueous solution containing at least one cation is saturated with cations by a continuous addition of hydroxide of said cation.

8. The method according to one of claims 1 to 7, **characterized in that** the pH of the aqueous solution containing at least one anion is comprised between 7 and 11.

9. The method according to one of claims 1 to 8, **characterized in that** step (f) of drying the mineralized biopolymer membrane is carried out at a temperature below 35°C, preferably for 12 to 24 hours.

10. A biopolymer membrane mineralized on both faces with nanocrystals of mineral compounds organized in the form of platelets and spherical nanoporous crystals having a honeycomb structure, obtainable by the method according to one of claims 1 to 9.

11. The membrane according to claim 10, **characterized in that** the biopolymer is collagen.

12. The membrane according to one of claims 10 to 11, **characterized in that** the nanocrystals consist of calcium phosphate.

13. A powder consisting of a mineralized biopolymer membrane according to one of claims 10 to 12, reduced to powder.

14. A solution comprising a powder according to claim 13 suspended in a pharmaceutically acceptable vehicle.

15. The mineralized biopolymer membrane according to one of claims 10 to 12, or powder according to claim 13, or solution according to claim 14, for the therapeutic use thereof in the treatment of bone, cartilaginous, pancreatic, renal, urethral, ureteral and vesical, testicular, ovarian, intestinal, hepatic, neurological, cardiac, tympanic, ocular, urinary, gynecological, pulmonary, bronchial, tracheal, vascular, connective, cutaneous, mucous, dental, gingival and/or hematopoietic and immune tissue disorders.
